# EUROPEAN PATENT SPECIFICATION

(11) **EP 2 966 441 B1**
(45) Date of publication and mention of the grant of the patent: **26.07.2017**
(21) Application number: 15275162.4
(22) Date of filing: 25.06.2015
(51) Int. Cl.: G01N 33/12, G01N 27/327, G01N 27/49, C12Q 1/00, C12Q 1/32

(54) **SENSOR AND METHOD FOR DETECTING ANDROSTENONE OR SKATOLE IN BOAR TAINT**
SENSOR UND VERFAHREN ZUM NACHWEIS VON ANDROSTENON SOWIE SKATOL IN EBERGERUCH
CAPTEUR ET PROCÉDÉ DE DÉTECTION DE L'ANDROSTÉNONE ET DU SCATOLE DANS L'ODEUR DE VERRAT

(30) Priority: 08.07.2014 GB 201412136
(43) Date of publication of application: 13.01.2016
(73) Proprietor: University of The West of England, Bristol, Bristol BS16 1QY (GB)
(72) Inventor: HART, John, Bristol, BS16 1QY (GB); CREW, Adrian, Bristol, BS16 1QY (GB); MCGUIRE, Natasha, Bristol BS5 9LP (GB); DORAN, Olena, Bristol, BS16 1QY (GB)
(74) Representative: Swan, Elizabeth Mary

(56) References cited:
- WO-A1-99/19507
- US-A- 4 791 057
- MUNDACA R A ET AL: "Enzyme biosensor for androsterone based on 3[alpha]-hydroxysteroid dehydrogenase immobilized onto a carbon nanotubes/ionic liquid/NAD+composite elect", TALANTA, vol. 99, 16 December 2012 (2012-12-16), pages 697-702, XP028936756, ISSN: 0039-9140, DOI: 10.1016/J.TALANTA.2012.07.008
- YI-WEI LI ET AL: "Recent advances in the dehydrogenase biosensors based on carbon nanotube modified electrodes", CHINESE JOURNAL OF ANALYTICAL CHEMISTRY, vol. 42, no. 5, May 2014 (2014-05), pages 759-765, XP055225398, ISSN: 1872-2040, DOI: 10.1016/S1872-2040(13)60733-1
- S. KOIDE ET AL: "Development of a micro-planar amperometric bile acid biosensor for urinalysis", BIOSENSORS AND BIOELECTRONICS, vol. 22, no. 9-10, 12 October 2006 (2006-10-12), pages 2079-2085, XP055225535, ISSN: 0956-5663, DOI: 10.1016/j.bios.2006.09.009
- MAESA J-M ET AL: "Voltammetric discrimination of skatole and indole at disposable screen printed electrodes", THE ANALYST, vol. 138, no. 5, 28 January 2013 (2013-01-28), page 1346, XP055225490, ISSN: 0003-2654, DOI: 10.1039/c3an36421f

## Description

### Field of the Invention

The present invention is concerned with a sensor array system for and a method of detecting, and preferably quantifying, androstenone and skatole, the chemicals associated with boar taint. The system can be used to detect and quantify boar taint in pig carcasses or live pigs and so can be used to prevent the entrance of tainted carcasses into the food chain and to allow the grading of carcasses as "premium quality".

### Background to the Invention

The term "boar taint" refers to a strong unpleasant odour and taste of pork and pork products which mainly affect male pigs. The odour and taste of meat with boar taint has a strong negative impact on consumer's perception of pork. Since pork is one of the most consumed meats in Europe, and in many other parts of the world, the pig industry represents a large and important part of the global economy. The problem of boar taint represents a major challenge to the global pig industry and finding a solution to this problem is of importance to the global economy.

It is known that boar taint is related to excessive accumulation of two main compounds, skatole (3-methyl-indole) and androstenone (5α-androst-16-en-3-one) in the subcutaneous adipose tissue of pigs. Skatole is produced from the amino acid tryptophan by bacterial action in pig gut and androstenone is produced in the testis in parallel with biosynthesis of other steroids. High skatole levels can be found not only in male pigs but also in some sows. In many countries threshold levels have been set, above which pork is deemed unsuitable for human consumption. For skatole and androstenone these threshold levels are 0.2-0.25ppm and 0.5-1 ppm respectively. The threshold levels vary between different countries.

Boar taint can generally be prevented by castration which has, to date, been the most popular solution to the problem. However castration has been discontinued in an increasing number of countries because of animal welfare issues. There is currently an ongoing debate in the European Union (EU) regarding possible alternatives to surgical castration and consequently the need for methods for detection of boar taint.

One alternative to castration is to raise entire (non-castrated) male pigs. This not only has animal welfare benefits but also has environmental and production benefits. For example, entire male pigs have been found to have a better feed conversion rate, higher growth rate and lean percentage, which reduces production costs. Entire male pigs also excrete less nitrogen when compared with castrates because of a better efficiency for nitrogen retention and so are more environmentally friendly. However, raising entire male pigs is associated with an increased risk of boar taint.

The risk of boar taint can be partially overcome by slaughter of the animals at an earlier age and lower weight than castrated males. However slaughtering at lower weight is inevitably associated with commercial losses. This is a particular issue for sectors of the industry which produce special types of high quality and high price ham (for example dry cured ham) which requires heavy weight pigs with defined meat quality. Therefore, if the industry is to move toward production of entire male pigs (especially heavy weight pigs), it has to ensure that the carcasses from these animals are boar taint free.

A second alternative to surgical castration is immunocastration which is subject to ongoing debate in the EU and has not been widely implemented. If immunocastration is to be widely implemented, an online method for evaluation of the effectiveness of immunocastration (i.e. skatole and androstenone levels in the meat) will still be required.

A third alternative to castration could be genetic selection toward boar-taint free pigs. Extensive work has been carried out in this area but several difficulties have been encountered. There is no genetic test for boar taint available at the present time and even if such a test becomes available in the future, there would still be a requirement for evaluating the effectiveness of genetic selection using a test for boar taint.

The level of one of the boar taint compounds, namely skatole, can be reduced by dietary manipulation and by a suitable management system. However, this approach does not allow regulation of the level of the other boar taint compound, androstenone. The effectiveness of dietary manipulation of skatole levels depends on the age and breed of animals. Therefore, a rapid method for evaluating the effectiveness of dietary manipulation in meat, and ideally in live animals, is required.

For the reasons discussed above, it is clear that whatever approach is chosen to reduce boar taint, it is essential to have an efficient method for evaluating skatole and/or androstenone levels in carcasses and meat products. It is preferable that any monitoring system is rapid, precise and low cost. Ideally the method would allow simultaneous or parallel detection of both skatole and androstenone and give skatole and androstenone levels in absolute values (rather than simply detect the presence) because the boar taint threshold differs between different countries. Such a monitoring system could be used to prevent the entrance of tainted carcasses into the food chain and to allow the grading of carcasses as "premium quality". Such a method is not currently available.

Indeed, the Scientific Panel on Animal Health and Welfare from the European Food Safety Authority declared that "there are no harmonised methods for consistently identifying carcasses with boar taint in commercial slaughter houses. Investigation of possible processing techniques to reduce the offensive properties of boar taint is hampered by the lack of such methods to assess levels of the compounds contributing to the phenomenon" (EFSA, 2004).

Various ways of measuring the concentration of skatole and/or androstenone have been proposed, which can be summarised briefly as follows.

A colorimetric method has been proposed for measuring skatole (see Mortensen, A. B. and Sorensen, S. E. (1984) Proceedings of the 30th European Meeting of Meat Research Workers, Bristol, Uk; AMSA; 9-14 September.) However, this method is seriously limited because it is non-specific, measures a range of compounds containing the indole group, requires a high level of operator competence, involves sub-sampling of meat online, and the presence of chemicals and optical equipment situated close to the meat processing facility. This method has not been adopted by the industry. Similarly colorimetric methods have been proposed for measuring the concentration of androstenone (see Squires, E. J. (1990) Canadian Journal of Animal Science 70: 1029-1040). This method also involves a long and expensive procedure involving the use of sample extraction, liquid chromatography and heating followed by colorimetric analysis. Hence, these methods are neither cost-effective nor quick enough for practical applications.

Various high technology analytical techniques have been suggested for measuring skatole and androstenone including high resolution gas chromatography (HRGC), high performance liquid chromatography (HPLC), gas chromatography (GC), GC-mass spectrometry and supercritical fluid chromatography/mass spectrometry (see Dehnhard, M., Claus, R., Hillenbrand, M. and Herzog, A. (1993) Journal of Chromatography: Biomedical Applications 616(2): 205-209; Hansen-Møller, J. (1994) Journal of Chromatography B: Biomedical Sciences and Applications 661(2): 219-230; Rius, M. A., Hortós, M. and Garcia-Regueiro, J. A. (2005) Meat Science 71(4): 595-602; Claus, R., Herbert, E. and Dehnhard, M. (1997) Archiv für Lebensmittelhygiene 48: 27-30; de Brabander, H. F. and Verbeke, R. (1986) Journal of Chromatography A 363(2): 293-302; Mågård, M. Å., Berg, H. E. B., Tagesson, V., Järemo, M. L. G., Karlsson, L. L. H., Mathiasson, L. J. E., Bonneau, M. and Hansen-Moller, J. (2002) Journal of Agricultural and Food Chemistry 43(1): 114-120; and Tuomola, M., Hakala, M. and Manninen, P. (1998) Journal of Chromatography B: Biomedical Sciences and Applications 719(1-2): 25-30). However, these methods are time-consuming because there are laborious sample preparation steps (samples have to be acquired, transported to a location remote from the pigs or carcasses and cleaned), only one sample can be analysed at a time and the methods are expensive as they require highly specialised costly equipment.

An "electronic nose" has been proposed for detecting skatole (see Annor-Frempong, I. E., Nute, G. R., Wood, J. D., Whittington, F. W. and West, A. (1998) Meat Science 50(2): 139-151) but this technology is limited largely because of questions surrounding its reliability and sensitivity and the high cost.

Various methods involving biological materials have been proposed for detecting androstenone including a radioimmunoassay, and enzyme-linked immunosorbent assay (ELISA) and a microtiter plate enzyme-immunoassay (see Claus, R. (1974) Dosage radioimmunologique du 5α-androst-16-en-3-one Comptes Rendus Hebdomadaire des Séances de l'Académie des Sciences Ser. D 278: 299-302; Andresen, Ø. (1975) Acta Endocrinological 79: 619-625; and Claus, R., Mahler, G. and Münster, E. (1988) Archiv für Lebensmittelhygiene 4: 87-90 respectively). However, these methods require antibodies and, to date, the antibodies that have been produced are not specific to androstenone and have significant cross-reactivity with other similar steroid compounds. In addition, the radioimmunoassay requires substances that are a danger to human health and clearly cannot be used in a non-laboratory setting. These methods are expensive and require high-cost equipment and facilities.

Finally, thickness shear mode resonator technology (TSMR) has also been proposed (see Di Natale, C., Pennazza, G., Macagnano, A., Martinelli, E., Paolesse, R. and D'Amico, A. (2003) Sensors & Actuators B 91(1-3): 169-174) but this is very non-specific and detects a range of other steroids. It is also very time-consuming and requires expensive specialised equipment.

It is clear that an urgent need exists for an improved method of detecting androstenone and/or skatole which are associated with boar taint. The present invention aims to provide a method of detecting androstenone and/or skatole that overcomes problems associated with existing methods. In particular the invention aims to provide a method of detecting, preferably quantifying, androstenone and/or skatole that is rapid, precise and low cost.

### Summary of the Invention

According to a first aspect, the present invention relates to an array of sensors as recited in claim 1.

According to a second aspect, the present invention relates to a method of detecting androstenone and skatole in a live animal, an animal carcass or a meat product, as recited in claim 10. The present invention is based on the realisation that electrochemical or optical methods can be used to provide improved methods for detecting and monitoring the chemicals that are associated with boar taint. In particular, the sensors for use in the present invention can detect androstenone and skatole in a rapid, precise and low cost way.

The present invention allows simultaneous or parallel detection of both skatole and androstenone and can give skatole and androstenone levels in absolute values, which is important because of the different boar taint thresholds in different countries. The present invention also provides for the first time a practical way of detecting the presence and monitoring the concentration of androstenone and skatole directly in carcasses, meat or even live animals, without the need for sample preparation.

In the case of androstenone, the invention makes use of the fact that certain enzymes can convert androstenone (5α-androst-16-en-3-one) to androstenol (5α-androst-16-en-3-ol) and that this conversion is dependent on the presence of a cofactor NAD(P)H. In particular, it was demonstrated that 3β-hydroxysteroid dehydrogenase was associated with the metabolism of androstenone in the liver of a pig (see Doran E,, Whittington F.M., Wood J.D. and McGiven J.D., Chemico-Biological Interactions 147 (2004) 141-149).

US 4 791 057 A describes a highly sensitive assay method for determining a 3-hydroxysteroid or 3-ketosteroid in a specimen using NAD(P).

TALANTA, vol. 99, 16th December 2012, pages 697-702, XP028936756, ISSN 0039-9140 describes an enzyme biosensor for detecting androsterone based on 3α-hydroxysteroid hydrogenase immobilised on carbon nanotubes/ RTIL/ NAD+ composite electrode. Hence, the sensor of the first aspect of the invention includes NADH or NADPH and 3-hydroxysteroid dehydrogenase, as well as means for detecting a change in the concentration of NADH or NADPH, the means comprising at least one electrode or an optical device.

NADH and NADPH refer to the reduced form of nicotinamide adenine dinucleotide and nicotinamide adenine dinucleotide phosphate respectively, which are well known in the art. Both NADH and NADPH act in the same way in the invention so either can be used. By NAD(P)H we mean either NADH or NADPH. The amount of NAD(P)H used is variable and depends on the electrode, enzyme activity on the electrode surface and the concentration of androstenone that is to be analysed. Typically amounts would be in the µg range.

The enzyme used in the sensor is 3-hydroxysteroid dehydrogenase, commonly referred to as HSD. When this enzyme comes into contact with androstenone, the androstenone is metabolised by the enzyme in a redox reaction in which the androstenone is reduced to androstenol and the NAD(P)H is oxidised to NAD(P)⁺. Two forms of the 3-HSD are known (α and β) and both can be used in the sensor of the invention. The α-form is preferred because it is commercially available, e.g. from Sigma-Aldrich Ltd, Diazyme Europe Gmbh, whereas the β-form requires specialized synthesis, e.g. by Abnova.

In the method of the invention, any enzyme that works in the same way as 3-hydroxysteroid dehydrogenase can be used i.e. any enzyme that metabolises androstenone in the presence of NADH or NADPH.

Sensors that include hydroxysteroid dehydrogenase have been suggested before in different contexts, see WO/99/19507 and Koide et al, Biosensors and Bioelectronics 22 (2007) 2079-2085. The sensors in these documents are not suggested as being of use with androstenone and, indeed, would be unsuitable for use in detecting androstenone. The sensors are substantially different to those of the present invention, at least in that they involve the initial presence of NAD(P)⁺ rather than the reduced form NAD(P)H.

The sensor also includes means for detecting a change in the concentration of NADH or NADPH, the means comprising at least one electrode or an optical device. As the androstenone is metabolised by the enzyme in the redox reaction, NADH or NADPH is converted to the oxidised form, NAD⁺ or NADP⁺ discussed above. Hence, by detecting a change in the NADH or NADPH concentration, the sensor indirectly detects the presence of androstenone, or another analyte that is metabolised by 3-hydroxysteroid dehydrogenase in the presence of NADH or NADPH.

In one embodiment, an optical device is used to detect a change in the concentration of NADH or NADPH. For example a combined system may be used comprising a source of a suitable wavelength of light and a detector (e.g. a spectrometer) to measure any absorbance of this light by the cofactor (NADH or NADPH) which could be housed in a transparent vessel/cuvette. The change in absorbance can be related to the concentration of androstenone. The concentration of androstenone could also be detected by means of the fluorescence of the cofactor. In both cases the wavelength of light to be absorbed is 340 nm.; For fluorescence measurement light emitted at 450 nm could be detected.

In the preferred embodiment, the means for detecting a change in the concentration of NADH or NADPH comprises at least one electrode. In this embodiment the sensor uses electrochemistry to detect the analyte. In this embodiment, the sensor preferably comprises at least two electrodes, a working electrode and a counter/reference electrode, more preferably three electrodes, a working electrode, a counter electrode and a reference electrode. Examples of electrode configurations that have been used for other biosensor applications by the inventors can be found in other publications by the inventors (see Hart, J. P., Crew, A., Crouch, E., Honeychurch, K. C. and Pemberton, R. M. (2004) Analytical Letters 37(5): 789-830 and Hart, J. P., Crew, A., Crouch, E., Honeychurch, K. and Pemberton, R. M. (2007) Comprehensive Analytical Chemistry. S. Alegret and A. Merkoci. Amsterdam, Elsevier B.V. 49: Chapter 23, 495-556).

The electrodes can be made of any suitable material known in the art. The electrodes can comprise metals, for example gold, platinum and silver. In a preferred embodiment, the electrodes comprise a carbon material including, for example, glassy carbon. The electrodes can be in any form, with suitable forms being well known to the person skilled in the art of electrochemistry. However, it is preferred that the electrodes are screen-printed. They can be screen-printed metals or carbon electrodes. Screen-printed electrodes are preferred because they are very low-cost to produce and convenient to use reproducible, disposable (lower contamination risk, and safe to use by the operator and in a food production environment), and readily fabricated in a wide range of geometries, e.g. squares, rectangles and discs. It is particularly preferred to use screen-printed carbon electrodes (SPCEs) as these may be even cheaper and more convenient.

The electrodes could be fabricated by screen-printing a commercially available carbon ink with the addition of the electron mediator either within the ink or onto the surface of the SPCE. The substrate containing the SPCEs could be one of many inert substrates, e.g. PVC or a ceramic material. In one design, the working carbon electrode area is defined by an insulating layer, and an Ag/AgCl counter/reference electrode is arranged around the working electrode in a 'hockey-stick' format. The screen-printing carbon ink suitable for this purpose can be obtained from Gwent Electronic Materials as well as carbon inks from other suppliers.

The NADH or NADPH and the 3-hydroxysteroid dehydrogenase can be used in the sensor in a solution that contacts the electrodes. However, in a preferred embodiment, the NADH or NADPH and the 3-hydroxysteroid dehydrogenase are immobilised on at least one of the electrodes, preferably on the working electrode. Immobilisation can take several forms, including drying the cofactor/enzyme onto the surface of the electrode, entrapping them in a membrane onto the surface of the electrode, or chemically cross-linking them directly to the electrode surface (working electrode only in all cases). In any enzyme-based sensor it is advantageous to minimize enzyme and cofactor use (very expensive) whilst maximising the signal generated at the electrode surface.

In the embodiment which involves at least one electrode to detect changes in the concentration of NAD(P)H, a redox reaction occurs at the electrode which allows detection of the concentration of NAD(P)H. In one embodiment, oxidation of NAD(P)H directly at the electrode surface to produce NAD(P)⁺. Hence, when some of the NAD(P)H is removed (due to a redox reaction with and analyte that is metabolised by the enzyme), the current from oxidation of the NAD(P)H at the electrode is decreased.

However, it is preferred that the sensor additionally comprises a redox mediator. Using a redox mediator is advantageous as it means that low voltages can be used This is in itself a very significant advantage and potentially enables the avoidance of signals from many interfering compounds. An additional advantage is that sensitivity can be improved. One such redox mediator is Meldola's Blue, which can be integrated into a screen-printing ink and deposited onto a substrate to produce the desired geometry of the sensor. Other mediators which can be used in the above reaction scheme include phenoxazines and phenazines as well as other similar classes of redox species which can be operated at low applied potentials. Other suitable mediators which could be utilised in the present invention are disclosed in WO99/19507. See also CHINESE JOURNAL OF ANALYTICAL CHEMISTRY, vol. 42, no. 5, May 2014, pages 759-765, XP055225398, ISSN 1872-2040, which discloses dehydrogenase biosensors using carbon nanotube (CNT) modified electrodes. In particular, it discusses the role of mediators, such as Meldola's Blue in overcoming the high overpotential for NADH oxidation at such electrodes. Hence, a preferred example of fabricating a NAD(P)H detection system is to use a water-soluble mediator, e.g. Meldola's Blue, which is prepared in an aqueous solution to be mixed with a carbon formulation. This is then printed onto the substrate and allowed to dry. The biosensor is then produced by immobilising HSD and NAD(P)H onto the surface of the working electrode or within the working electrode of the SPCE.

Whether or not a mediator is used a potential difference must be applied across the electrodes and the system must be interrogated to determine the current arising from the redox reactions occurring. The sensor of the present invention preferably includes apparatus (e.g. a potentiostat) to apply the potential difference and to interrogate the electrodes. A wide variety of electrochemical techniques could be employed for interrogating the systems described above; for example (but not restricted to), cyclic voltammetry, sampled DC voltammetry, pulse voltammetry and its variants including square-wave voltammetry, chronoamperometry and amperometry in stirred solution. The voltage required is dependent on the species, pH and type of electrode; for example for the oxidation of NAD(P)H using the Meldola's Blue -SPCE the voltage may typically be +50 mV when using amperometry. Using a different technique, for example linear sweep voltammetry, a range of potentials e.g. -300 mV to +500 mV could be involved.

According to the first aspect of the invention, an array of sensors is provided including a sensor for androstenone and a sensor for detecting skatole comprising at least one electrode. In this way the present invention allows simultaneous or parallel detection, preferably quantification, of both skatole and androstenone.

There are possible arrangements for the combination of the two sensors into the same device, which could be used for detecting boar taint. For example, the device could comprise: an on-line device where a sample or extract is taken via an invasive probe and presented to the sensors (individually or in combination); an on-line device where the sensors (individually or in combination) are presented to the material subject to the analysis directly in an invasive probe; or a device for transcutaneous measurement on live animals.

The skatole (3-methyl-indole) sensor detects the presence of skatole by direct oxidation of the skatole at the surface of the electrode. As for the androstenone sensor, the electrodes can be made of any suitable material known in the art such as gold, platinum, silver and carbon and can be in any form. It is preferred that the electrodes are screen-printed, and it is particularly preferred to use screen-printed carbon electrodes.

In a preferred embodiment, the sensor for skatole comprises a screen-printed working electrode (3x3mm) surrounded by a screen-printed Ag/AgCl counter/reference electrode. The current response for skatole arises from direct oxidation at the surface of the screen-printed carbon electrode (SPCE).

With regard to the geometric shape of the sensor, a design reported previously to measure alcohol could be used, see Sprules, S. D., Hartley, I. C., Wedge, R., Hart, J. P. and Pittson, R. (1996b) Analytica Chimica Acta 329(3): 215-221.

The measurement of skatole can be carried out in a buffer solution at an optimised pH and buffer salt composition. The sample can be interrogated in several ways including the following:
The electrode is placed into the optimised buffer solution containing the macerated meat/tissue sample;
supernatant can be deposited onto the surface of the skatole sensor; or
by direct insertion of the skatole sensor into the meat/tissue of the carcass/meat product.

The sensor for detecting skatole preferably additionally comprises apparatus for interrogating the sensor to detect oxidation of skatole at the surface of at least one electrode. As for androstenone; a potentiostat is required. The potential depends on several factors e.g. pH, membrane type, In one example we have used linear sweep voltammetry and scanned the voltage from -0.5 V to +1.5 V. The signals appear in the range +0.5 V to + 1.5 V. A wide variety of electrochemical waveforms can be employed, for example, cyclic voltammetry, sampled DC voltammetry, pulse voltammetry and its variants including square-wave voltammetry, chronoamperometry and amperometry in stirred solution.

THE ANALYST, vol. 138, no. 5, 28th January 2013, page 1346, XP055225490, ISSN 0003-2654 discloses the voltammetric determination of skatole as a marker for boar taint in real samples. The array of sensors of the present invention is used for detecting androstenone and skatole in a live animal, an animal carcass or a meat product. Hence, the sensor can be inserted directly into a live animal, an animal carcass or a meat product, preferably wherein the animal is a pig, most preferably wherein the sensor is inserted into the subcutaneous adipose tissue of a pig. Another embodiment of the present invention relates to a method of contacting the sensor with the live animal, a sample from the live animal, the animal carcass, a sample from the animal carcass, the meat product or a sample from the meat product. An advantage of the present invention compared to prior art methods is it that it can be used directly on a live animal, an animal carcass or a meat product. This represents a significant improvement over the need to take samples and analyse them remotely, although samples can also be analysed in the present invention. Accordingly, the array of sensors is used directly on a live animal, an animal carcass or a meat product in the form of a probe which is suitable for insertion into the live animal, animal carcass or meat product. Several approaches are possible. One device may allow contact between the sensor surface and the tissue of the animal; this could be a sharp ended device which allows easy penetration into the carcass. This device could have dimensions, for example, of width 0.5-1.5cm and length 1-2cm. However, these dimensions can dramatically be reduced by using microelectrodes which are in the range 50-100 µm. The front of the probe, (which may house the sensors, would be inserted into the subcutaneous fat to allow direct measurement of the two boar taint compounds. The probe may take the form of either a solid device with the sensors on an external surface which enables the sensors to make contact with the tissue directly as it is inserted, or a device that contains the sensors within a protective housing that allows contact between the sensors and the tissue via an opening at the end. The latter could be operated in one of several ways; e.g. biological fluid could be drawn into the probe either actively or passively for interrogation; the former may require a piston type of device.

### Figures

Methods and sensor arrangements in accordance with the invention will now be further described by way of example only, with reference to the accompanying drawings, Figures 1-13.
Figure 1 shows a reaction scheme for androstenone at modified screen-printed carbon electrodes (SPCE), where Mₒₓ and M_{red} are oxidised and reduced forms of the mediator;
Figure 2 shows the format of the screen-printed carbon electrodes comprising Meldola's Blue (MB-SPCE) connected by gold clips to a potentiostat;
Figure 3 shows the current response of MB-SPCEs to additions of 5×10⁻⁵ M of NADH added to free solution of 50mM phosphate buffer pH7; applied potential +50V vs Ag/AgCl;
Figure 4 shows the current response of MB-SPCEs to additions of 1×10⁻⁵ M of androstenone added to free solution of 5×10⁻⁵ M NADH with 10U HSD in 50mM phosphate buffer pH7; applied potential +50V vs Ag/AgCl;
Figure 5 shows the chronoamperometric response of MB-SPCEs to NADH added to free solution of 50mM phosphate buffer pH7;
Figure 6 shows the chronoamperometric response of MB-SPCEs to androstenone added to free solution of 5×10⁻⁵ M NADH with 10U 3α-HSD in 50mM phosphate buffer pH7;
Figure 7 shows the chronoamperometric data for biosensor comprising 2U HSD with 60µg NADH in a solution of 50mM phosphate buffer pH7 and 0.1M NaCl with either 0, 1 or 5µM androstenone;
Figure 8 shows the chronoamperometric calibration of the HSD biosensors with androstenone. Data taken from current values at 240 and 300 seconds; individual biosensors were used for each concentration;
Figure 9 shows the direct electrochemical oxidation of skatole (3-methyl-indole) that can take place at the surface of a SPCE.
Figure 10 shows cyclic voltammetry using a D14-SPCE of 0.5mM skatole in pH8 buffer.
Figure 11 shows a calibration plot for skatole using amperometry in stirred solution with a D14-SPCE.
Figure 12 shows the results of direct analysis of androstenone in adipose tissue.
Figure 13 shows the results of direct analysis of skatole in adipose tissue.

### Detailed Description of the Invention

Preferred embodiments of the invention are now described with reference to the figures.

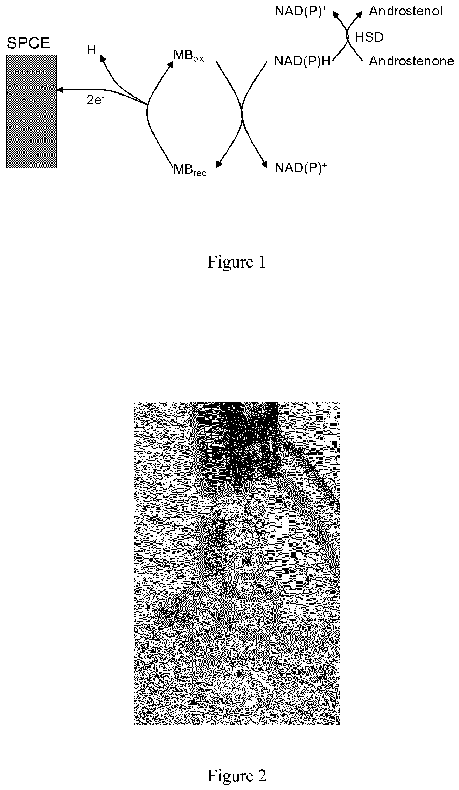

Figure 1 shows a reaction scheme for androstenone at modified screen-printed carbon electrodes (SPCE), where Mₒₓ and M_{red} are oxidised and reduced forms of the mediator. In this system the analytical response arises from the electrocatalytic oxidation of NAD(P)H which involves a redox mediator (Mₒₓ/M_{red}). The maximum electrocatalytic current occurs in the absence of androstenone; in the presence of this analyte some NAD(P)H is removed from the pool on the electrode surface, consequently, the electrocatalytic signal decreases. This decrease in response is proportional to the concentration of androstenone ion present in the sample.

The above reaction scheme could also operate in the absence of the electron mediator where oxidation occurs directly at the electrode surface.

### Fabrication & Evaluation of the Electrochemical NAD(P)H Sensor

An example of the electrochemical measurement of NADH (but could incorporate NADPH) in solution has been demonstrated by the inventors. The screen-printed carbon electrodes (SPCE) containing the Meldola's Blue (MB-SPCE) was produced. The MB-SPCE comprised a water-based carbon electrode with an active area of 3×3mum defined by an insulation layer. This was fabricated by screen-printing an ink prepared by mixing an aqueous Meldola's Blue solution with the appropriate quantity of the base ink components. An Ag/AgCl counter/reference electrode was arranged around the working electrode in a 'hockey-stick' format (see Figure 2 which shows the format of the MB-SPCE connected by gold clips to a potentiostat).

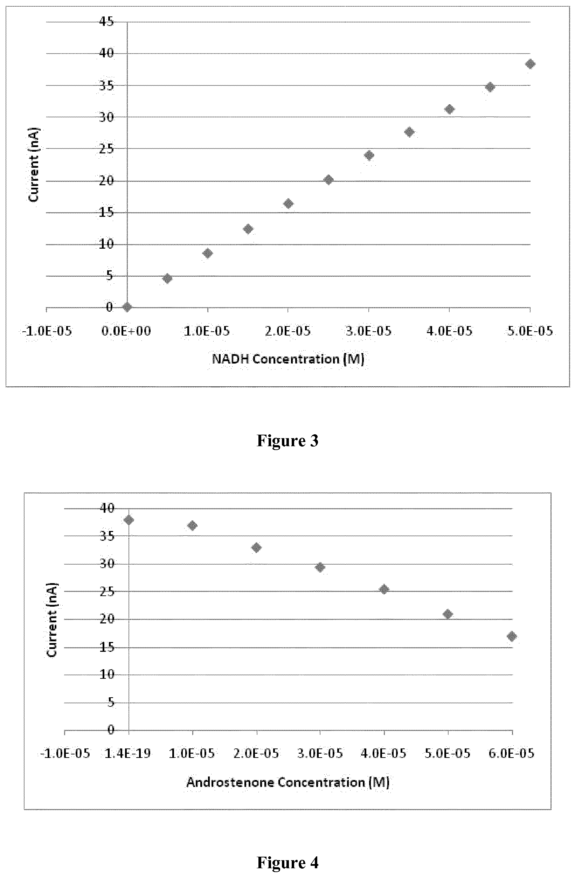

Following the fabrication of the transducer, initial studies were performed using amperometry in stirred solution to demonstrate the feasibility of exploiting the electrocatalytic oxidation of NADH to optimise the assay. Figure 3 relates to the current response of MB-SPCEs to additions of 5×0⁻⁵ M of NADH added to free solution of 50mM phosphate buffer pH7; applied potential +50mV vs Ag/AgCl. Figure 3 shows that a linear relationship exists between the steady state current responses and concentration of NADH. This concentration range encompasses the range that is required for the biosensor operation, therefore showing that the MB-SPCEs are capable of measuring NADH in the desired concentration range.

There was no current response with the addition of the enzyme into solution, demonstrating that there was no interaction between the cofactor and 3α-HSD. The response of MB-SPCEs to additions of androstenone into solutions containing NADH together with HSD was then examined. Figure 4 relates to the current response of MB-SPCEs to additions of 1×10⁻⁵ M of androstenone added to free solution of 5×10⁻⁵ M NADH with 10U HSD in 50mM phosphate buffer pH7; applied potential +50mV vs Ag/AgCl. Figure 4 shows the reduction in current corresponding with additions of 10µM androstenone, which was caused by the removal of NADH during the conversion of androstenone to androstenol by HSD (as shown in Figure 1). This behaviour demonstrates that the combination of MB-SPCEs and the free enzyme/NADH can be used to determine androstenone concentration in solution.

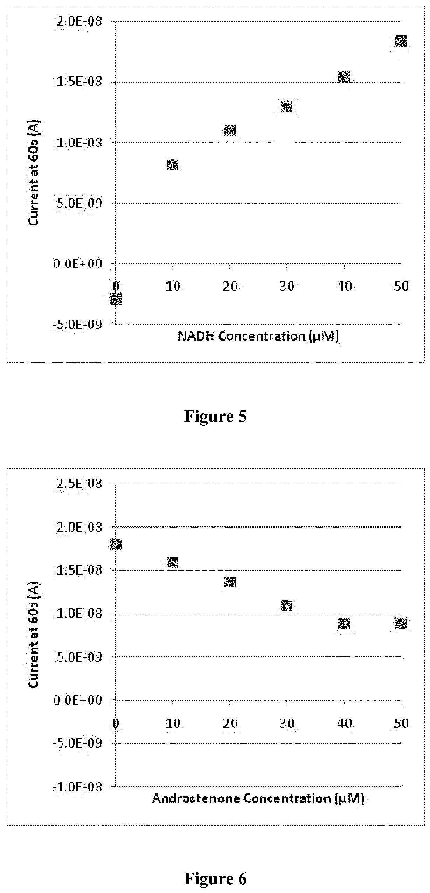

The sensors, together with solubilised reagents, were further examined using chronoamperometry, which is later discussed for the operation of the biosensors. In this technique the sensors are initially held at open circuit for a fixed time then a suitable oxidation potential is applied which results in the current response; this is measured over a defined time period. Figure 5 relates to the chronoamperometric response of MB-SPCEs to NADH added to free solution of 50mM phosphate buffer pH7. Figure 5 shows that the current responses were taken at 60 seconds and were found to increase in proportional to NADH concentration in the concentration range 0-50µM. Figure 6 relates to the chronoamperometric response of MB-SPCEs to androstenone added to free solution of 5×10⁻⁵ M NADH with 10U 3α-HSD in 50mM phosphate buffer pH7. Figure 6 shows that, conversely, the current response at 60 seconds was reduced with increasing androstenone concentration in the concentration range 0-50µM.

### Fabrication & Evaluation of the Electrochemical Androstenone Biosensor

Biosensors were produced by immobilising 3α-HSD and NADH (Sigma-Aldrich) (60µg) onto the surface of the MB-SPCE (immobilisation could be carried out by direct incorporation of the components directly into the ink). The decrease in the amperometric current produced was significantly greater with 2U of the enzyme immobilised onto the electrode surface. Therefore, 2U of HSD was used for further biosensor studies.

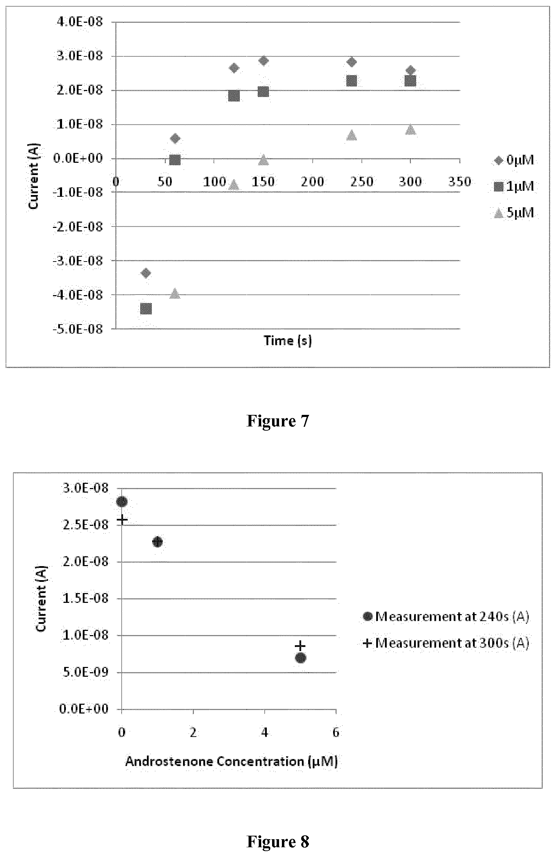

These biosensors were used to analyse buffered solutions containing 0, 1 or 5µM androstenone. These concentrations represent approximately the concentrations that a biosensor could be expected to analyse in an extract of adipose or other suitable samples of tissue for the detection of boar taint. The chronoamperograms are presented in Figure 7 which relates to the chronoamperometric data for biosensor comprising 2U HSD with 60µg NADH in a solution of 50mM phosphate buffer pH7 and 0.1M NaCl with either 0, 1 or 5µM androstenone. Figure 7 clearly shows the reduction in current at higher androstenone concentrations; this current difference is proportional to the analyte concentration.

In the above example, a stable current is obtained at 240 seconds, after which the reduction in current is proportional to the androstenone concentration. Figure 8 relates to the chronoamperometric calibration of the HSD biosensors with androstenone. Data taken from current values at 240 and 300 seconds; individual biosensors were used for each concentration. Indeed, the reduction in current at both 240 and 300 seconds is directly proportional to the androstenone concentration, as can be seen in Figure 8.

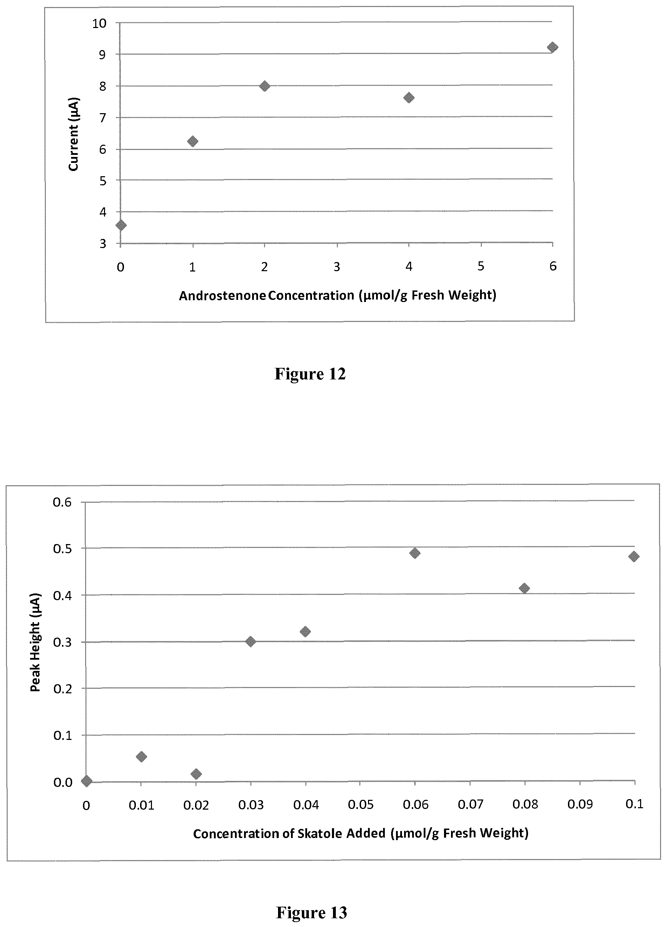

These biosensors were also used for the direct analysis of androstenone in adipose tissue. In this experiment, androstenone was spiked at each concentration into one of five subsamples on approximately 5-6g in weight. The androstenone was dissolved in methanol and introduced into the fat samples (a maximum of 10µl volume). After the methanol had evaporated, each sample was sealed in a sterile container for 24 hours prior to analysis. The analysis was performed by inserting the biosensor into an incision made in the tissue using a surgical scalpel. The results are given in Figure 12 and clearly show that an increased response was achieved with increasing androstenone concentration.

### Fabrication & Evaluation of Skatole Sensor

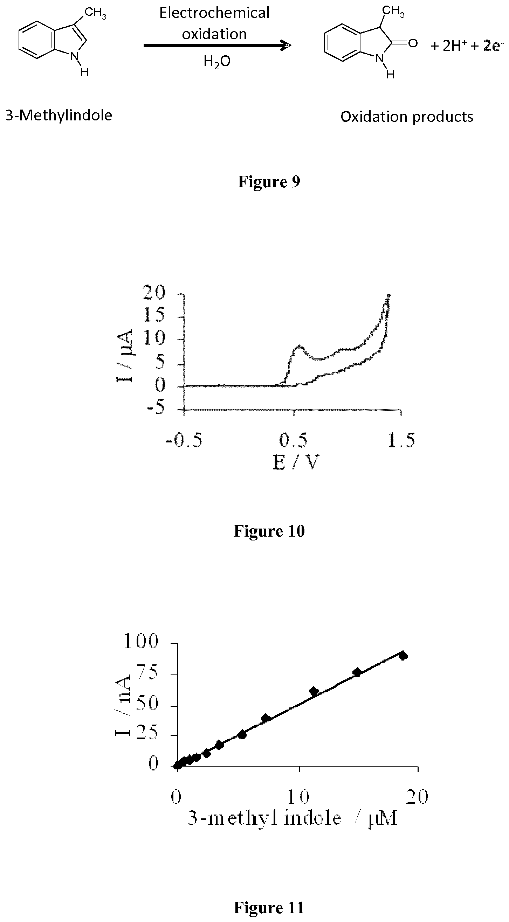

An example of the electrochemical measurement of skatole in solution has been demonstrated by the inventors. This involves the use of a screen-printed carbon electrode (SPCE) which is placed in an aqueous buffer solution containing skatole. The screen-printed working electrode (3x3mm) is surrounded by a screen-printed Ag/AgCl counter/reference electrode. The current response for skatole arises from direct oxidation at the surface of the screen-printed carbon electrode (SPCE) (See Figure 9).

Figure 10 shows a typical cyclic voltammetric response obtained with a SPCE using a solution comprising 0.5 mM skatole in pH 8.0 buffer solution. Clearly a well-defined response was obtained which was the result of an oxidation reaction (see Figure 9). Under optimised conditions it was also possible to measure skatole in the presence of indole. A calibration study was performed using the technique known as amperometry in stirred solution; the response for skatole was found to be linear over the concentration range studied 0.5-20 µm (Figure 11).

The fabricated skatole sensor was also used for the direct analysis of skatole in adipose tissue. This experiment was conducted in exactly the same way as the analysis of androstenone in adipose tissue, but with the use of the skatole sensor rather than the androstenone biosensor. The results are given in Figure 13 and show direct analysis of skatole is feasible throughout the concentration range necessary for the detection of boar taint.

## Claims

1. A sensor array for detecting androstenone and skatole in a live animal, a sample from the live animal, an animal carcass, a sample from the animal carcass, a meat product or a sample from the meat product, the sensor array comprising:
(a) a sensor for androstenone comprising NADH or NADPH; an enzyme that metabolises androstenone in the presence of NADH or NADPH; and means for detecting a change in the concentration of NADH or NADPH, the means comprising at least one electrode or an optical device; and
(b) a sensor for detecting skatole comprising at least one electrode;
**characterised in that** the sensor array is situated in or on a probe that can be contacted with the live animal, a sample from the live animal, the animal carcass, a sample from the animal carcass, the meat product or a sample from the meat product; and
the sensor array allows for simultaneous or parallel detection of both skatole and androstenone levels by detecting a change in the level of NADH or NADPH caused by metabolism of the androstenone using the at least one electrode or the optical device of sensor (a) and detecting skatole using the electrode of the sensor (b).

2. A sensor array according to claim 1, wherein the means for detecting a change in the concentration of NADH or NADPH comprises at least one electrode, preferably at least two electrodes, a working electrodes and a counter/reference electrode, more preferably three electrodes, a working electrode, a counter electrode and a reference electrode.

3. A sensor array according to claim 2, wherein the at least one of the electrodes comprises carbon, preferably wherein the working electrode comprises carbon, most preferably wherein the electrodes are screen-printed carbon electrodes.

4. A sensor array according to any of claims 1 to 3, wherein the enzyme that metabolises androstenone in the presence of NADH or NADPH is 3-hydroxysteroid dehydrogenase.

5. A sensor array according to claim 4, wherein the NADH or NADPH and the 3-hydroxysteroid dehydrogenase are immobilised on at least one of the electrodes, preferably wherein they are immobilised on the working electrode.

6. A sensor array according to any preceding claim, wherein the sensor (a) additionally comprises a redox mediator, preferably wherein the redox mediator is Meldola's Blue.

7. A sensor array according to claim 1, wherein the sensor for detecting skatole additionally comprises apparatus for interrogating the sensor with cyclic voltammetry, sampled DC voltammetry, pulse voltammetry, square-wave voltammetry, chronoamperometry or amperometry to detecting oxidation of skatole at the surface of the at least one electrode.

8. A sensor array according to claim 1 or 7, wherein the sensor for detecting skatole includes at least two electrodes, a working electrodes and a counter/reference electrode, more preferably three electrodes, a working electrode, a counter electrode and a reference electrode, wherein the at least one of the electrodes comprises carbon, most preferably wherein the electrodes are screen-printed carbon electrodes.

9. A sensor array according to any preceding claim, in the form of a probe for insertion into the subcutaneous adipose tissue of a pig.

10. A method of detecting androstenone and skatole in a live animal, a sample from the live animal, an animal carcass, a sample from the animal carcass, a meat product or a sample from the meat product, the method comprising the steps of:
(i) providing an array of sensors situated in or on a probe and comprising:
(a) a sensor for androstenone comprising NADH or NADPH; an enzyme that metabolises androstenone in the presence of NADH or NADPH; and means for detecting a change in the concentration of NADH or NADPH, the means comprising at least one electrode or an optical device; and
(b) a sensor for detecting skatole comprising at least one electrode;
the method **characterised by**
(ii) contacting the probe with the live animal, a sample from the live animal, the animal carcass, a sample from the animal carcass, the meat product or a sample from the meat product; and
(iii) detecting a change in the level of NADH or NADPH caused by metabolism of the androstenone, using the at least one electrode or the optical device of the sensor in (a) and detecting skatole using the electrode of the sensor (b).

11. A method according to claim 10, wherein the enzyme is 3-hydroxysteroid dehydrogenase.

12. A method according to claim 10 or 11, wherein the probe is contacted with a sample from an live animal or a carcass, preferably wherein the animal is a pig.

13. A method according to claim 12, wherein the probe is inserted into a live animal, an animal carcass or a meat product, preferably wherein the animal is a pig, most preferably wherein the sensor is inserted into the subcutaneous adipose tissue of a pig.

## Patentansprüche

1. Sensoranordnung zum Detektieren von Androstenon und Skatol in einem lebenden Tier, einer Probe aus dem lebenden Tier, einem Tierkadaver, einer Probe aus dem Tierkadaver, einem Fleischerzeugnis oder einer Probe aus dem Fleischerzeugnis, wobei die Sensoranordnung umfasst:
(a) einen Sensor für Androstenon umfassend NADH oder NADPH; ein Enzym welches Androstenon bei Anwesenheit von NADH oder NADPH metabolisiert; und Mittel zum Detektieren einer Änderung der Konzentration von NADH oder NADPH; wobei die Mittel mindestens eine Elektrode oder eine optische Einrichtung umfassen; und
(b) einen Sensor zum Detektieren von Skatol umfassend mindestens eine Elektrode;
**dadurch gekennzeichnet, dass** die Sensoranordnung in oder auf einer Sonde angeordnet ist, welche mit dem lebenden Tier, einer Probe aus dem lebenden Tier, dem Tierkadaver, einer Probe aus dem Tierkadaver, dem Fleischerzeugnis oder einer Probe aus dem Fleischerzeugnis kontaktiert werden kann; und
die Sensoranordnung ermöglicht eine gleichzeitige oder parallele Detektion sowohl von Skatol- als auch von Androstenon-Gehalten durch das Detektieren einer Änderung des Gehaltes von NADH oder NADPH verursacht durch den Metabolismus von Androstenon unter Verwendung der mindestens einen Elektrode oder der optischen Einrichtung des Sensors (a) und das Detektieren von Skatol unter Verwendung der Elektrode des Sensors (b).

2. Sensoranordnung nach Anspruch 1, wobei die Mittel zum Detektieren einer Änderung in der Konzentration von NADH oder NADPH mindestens eine Elektrode, vorzugsweise mindestens zwei Elektroden, eine Arbeitselektrode und eine Gegen-/Referenzelektrode, besonders bevorzugt drei Elektroden, eine Arbeitselektrode, eine Gegenelektrode und eine Referenzelektrode, umfassen.

3. Sensoranordnung nach Anspruch 2, wobei die mindestens eine der Elektroden Kohlenstoff umfasst, vorzugsweise wobei die Arbeitselektrode Kohlenstoff umfasst, besonders bevorzugt wobei die Elektroden Siebdruck-Kohlenstoffelektroden sind.

4. Sensoranordnung nach einem der Ansprüche 1 bis 3, wobei das Enzym, welches Androstenon in der Anwesenheit von NADH oder NADPH metabolisiert, 3-Hydroxysteroid-Dehydrogenase ist.

5. Sensoranordnung nach Anspruch 4, wobei das NADH oder NADPH und die 3-Hydroxysteroid-Dehydrogenase auf mindestens einem der Elektrode immobilisiert sind, vorzugsweise wobei sie auf der Arbeitselektrode immobilisiert sind.

6. Sensoranordnung nach einem der vorgehenden Ansprüche, wobei der Sensor (a) zusätzlich einen Redoxmediator umfasst, vorzugsweise wobei der Redoxmediator Meldola Blau ist.

7. Sensoranordnung nach Anspruch 1, wobei der Sensor zum Detektieren von Skatol zusätzlich eine Vorrichtung zum Abfragen des Sensors durch zyklische Voltammetrie, gesampelter Gleichstromvoltammetrie, Pulsvoltammetrie, Rechteckwellenvoltammetrie, Chronoamperometrie oder Amperometrie zum Detektieren von Oxidation von Skatol an der Oberfläche der mindestens einen Elektrode umfasst.

8. Sensoranordnung nach Anspruch 1 oder 7, wobei der Sensor zum Detektieren von Skatol mindestens zwei Elektroden umfasst, eine Arbeitselektrode und eine Gegen-/Referenzelektrode, bevorzugt drei Elektroden, eine Arbeitselektrode, eine Gegenelektrode und eine Referenzelektrode, wobei die mindestens eine von den Elektroden Kohlenstoff umfasst, besonders bevorzugt wobei die Elektroden Siebdruck-Kohlenstoffelektroden sind.

9. Sensoranordnung nach einem der vorgehenden Ansprüche, in der Form von einer Sonde zum Einführen in das subkutane Fettgewebe eines Schweins.

10. Verfahren zum Detektieren von Androstenon und Skatol in einem lebenden Tier, einer Probe aus dem lebenden Tier, einem Tierkadaver, einer Probe aus dem Tierkadaver, einem Fleischerzeugnis oder einer Probe aus dem Fleischerzeugnis, wobei das Verfahren die Schritte umfasst:
(i) Bereitstellen einer Sensoranordnung angeordnet in oder auf einer Sonde und umfassend:
(a) einen Sensor für Androstenon umfassend NADH oder NADPH; ein Enzym das Androstenon in der Anwesenheit von NADH oder NADPH metabolisiert; und Mittel zum Detektieren einer Änderung in der Konzentration von NADH oder NADPH, wobei die Mittel mindestens eine Elektrode oder eine optische Einrichtung umfassen; und
(b) einen Sensor zum Detektieren von Skatol umfassend mindestens eine Elektrode;
das Verfahren ist **gekennzeichnet durch**
(ii) Kontaktieren der Sonde mit dem lebenden Tier, einer Probe aus dem lebenden Tier, dem Tierkadaver, einer Probe aus dem Tierkadaver, dem Fleischerzeugnis oder einer Probe aus dem Fleischerzeugnis; und
(iii) Detektieren einer Änderung des Gehaltes von NADH oder NADPH verursacht durch den Metabolismus von dem Androstenon unter Verwendung der mindestens einen Elektrode oder der optischen Einrichtung des Sensors in (a) und Detektieren von Skatol unter Verwendung der Elektrode des Sensors (b).

11. Verfahren nach Anspruch 10, wobei das Enzym 3-Hydroxysteroid-Dehydrogenase ist.

12. Verfahren nach Ansprüche 10 oder 11, wobei die Sonde mit einer Probe aus einem lebenden Tier oder einem Tierkadaver kontaktiert wird, vorzugsweise wobei das Tier ein Schwein ist.

13. Verfahren nach Anspruch 12, wobei die Sonde in ein lebendes Tier, einen Tierkadaver oder ein Fleischerzeugnis eingeführt wird, vorzugsweise wobei das Tier ein Schwein ist, besonders bevorzugt wobei der Sensor in subkutanes Fettgewebe eines Schweins eingeführt wird.

## Revendications

1. Réseau de capteurs permettant de détecter la présence d'androsténone et de scatole chez un animal vivant, un échantillon d'animal vivant, une carcasse d'animal, un échantillon de carcasse d'animal, un extrait de viande ou un échantillon d'extrait de viande, ce réseau de capteurs comprenant :
(a) un capteur d'androsténone renfermant de la NADH ou des NADPH, une enzyme qui métabolise l'androsténone en présence de NADH ou de NADPH et des moyens permettant de détecter une variation de la concentration en NADH ou NADPH, ces moyens comprenant une électrode et/ou un dispositif optique, et
(b) un capteur de détection du scatole comprenant au moins une électrode,
**caractérisé en ce que**
le réseau de capteurs est situé dans ou sur une sonde qui peut être mise en contact avec l'animal vivant, un échantillon de l'animal vivant, la carcasse animale, un échantillon de la carcasse animale, l'extrait de viande ou un échantillon de l'extrait de viande, et
le réseau de capteurs permet une détection simultanée ou parallèle des taux de scatole et d'androsténone en détectant une variation du taux de NADH ou de NADPH provoquée par le métabolisme de l'androsténone en utilisant l'électrode ou le dispositif optique du capteur (a) et en détectant le scatole en utilisant l'électrode du capteur (b).

2. Réseau de capteurs conforme à la revendication 1,
dans lequel les moyens permettant de détecter une variation de la concentration en NADH ou en NADPH comprennent au moins une électrode, de préférence au moins deux électrodes, à savoir une électrode de travail et une électrode de référence contre électrode, et plus préférentiellement trois électrodes, à savoir une électrode de travail, une contre électrode et une électrode de référence.

3. Réseau de capteurs conforme à la revendication 2,
**caractérisé en ce que**
l'électrode renferme du carbone, de préférence l'électrode de travail renferme du carbone et de façon plus préférentielle, les électrodes sont des électrodes en carbone imprimées par sérigraphie.

4. Réseau de capteurs conforme à l'une quelconque des revendications 1 à 3,
dans lequel l'enzyme qui métabolise l'androsténone en présence de NADH ou de NADPH est la 3-hydroxystéroïde déshydrogénase.

5. Réseau de capteurs conforme à la revendication 4,
dans lequel la NADH ou le NADPH et la 3-hydroxystéroïde déshydrogénase sont immobilisés sur au moins l'une des électrodes, et de préférence sur l'électrode de travail.

6. Réseau de capteurs conforme à l'une des revendications précédentes,
dans lequel le capteur (a) renferme en outre un médiateur redox, le médiateur redox étant de préférence du bleu de Meldola.

7. Réseau de capteurs conforme à la revendication 1,
dans lequel le capteur de détection du scatole renferme en outre un appareil permettant d'interroger le capteur par voltamétrie cyclique, voltamétrie d'essai d'échantillon, voltamétrie d'impulsions, voltamétrie d'onde carrée, chronoampéromètre ou ampéromètrie pour détecter l'oxydation du scatole à la surface de l'électrode.

8. Réseau de capteurs conforme à la revendication 1 ou 7,
dans lequel le capteur de détection du scatole renferme au moins deux électrodes, à savoir une électrode de travail et une électrode de référence contre électrode, et de façon plus préférentielle, trois électrodes, à savoir une électrode de travail, une contre électrode et une électrode de référence, au moins l'une de ces électrodes renfermant du carbone, et de façon plus préférentielle, les électrodes étant des électrodes en carbone imprimées en sérigraphie.

9. Réseau de capteurs conforme à l'une quelconque des revendications précédentes se présentant sous la forme d'une sonde destinée à être insérée dans le tissu adipeux sous-cutané d'un pore.

10. Procédé de détection de l'andosténone et du scatole chez un animal vivant, un échantillon d'animal vivant, une carcasse d'animal, un échantillon de carcasse d'animal, un extrait de viande ou un échantillon d'extrait de viande comprenant des étapes consistant à :
(i) se procurer un réseau de capteurs situé dans ou sur une sonde et comprenant :
(a) un capteur d'andosténone renfermant de la NADH ou du NADPH, une enzyme qui métabolise l'andosténone en présence de NADH ou de NADPH et des moyens de détection d'une variation de la concentration en NADH ou NADPH, ces moyens comprenant une électrode et/ou un dispositif optique, et
(b) un capteur de détection du scatole comprenant au moins une électrode, procédé **caractérisé par**,
des étapes consistant à :
(ii) mettre en contact la sonde avec l'animal vivant, un échantillon d'animal vivant, la carcasse animale, un échantillon de carcasse animale, l'extrait de viande ou un échantillon d'extrait de viande, et
(iii) détecter une variation du taux de NADH ou de NADPH provoquée par le métabolisme de l'androsténone en utilisant l'électrode ou le dispositif optique du capteur (a) et détecter le scatole en utilisant l'électrode du capteur (b).

11. Procédé conforme à la revendication 10,
dans lequel l'enzyme est la 3-hydroxystéroïde déhydrogénase.

12. Procédé conforme à la revendication 10 ou 11,
selon lequel la sonde est mise en contact avec un échantillon d'animal vivant ou une carcasse, et de préférence l'animal est un porc.

13. Procédé conforme à la revendication 12,
selon lequel la sonde est insérée dans l'animal vivant, une carcasse d'animal ou un extrait de viande, l'animal étant de préférence un porc et de façon plus préférentielle, la sonde étant insérée dans le tissu adipeux sous-cutané d'un porc.
